# EUROPÄISCHE PATENTANMELDUNG

(11) **EP 2 388 580 A1**
(43) Veröffentlichungstag der Anmeldung: **23.11.2011**
(21) Anmeldenummer: 10162946.7
(22) Anmeldetag: 17.05.2010
(51) Int. Cl.: G01N 33/00, G01N 27/68

(54) **Vorrichtung und Verfahren zur Detektion eines durch eine elektrische Entladung erzeugtes Umwandlungsprodukt eines gasförmigen Schadstoffes**

(71) Anmelder: Airsense Analytics GmbH, 19061 Schwerin (DE)
(72) Erfinder: Walte, Andreas, Dr., 19059, Schwerin (DE); Münchmeyer, Wolf, 38468, Ehra-Lessien (DE)
(74) Vertreter: Gulde Hengelhaupt Ziebig & Schneider

(57) **Zusammenfassung**

Vorrichtung (16) zur Detektion gasförmiger Schadstoffe mit einer Einrichtung (22) zur Zerlegung der zu detektierenden gasförmigen Schadstoffes in wenigstens ein Umwandlungsprodukt (24) und einer Einrichtung (26) zur selektiven Bestimmung des wenigstens einen Umwandlungsproduktes (24).
Es ist vorgesehen, dass die Einrichtung (22) zur Zerlegung des zu detektierenden gasförmigen Schadstoffes wenigstens einen Reaktionsraum (28) umfasst, innerhalb dem eine elektrische Entladung (34) erzeugbar ist.
Ferner ein Verfahren zur Detektion gasförmiger Schadstoffe, wobei der gasförmige Schadstoff in wenigstens ein Umwandlungsprodukt (24) zerlegt wird. Hierzu ist vorgesehen, dass der zu detektierende gasförmige Schadstoff zur Zerlegung in das wenigstens eine Umwandlungsprodukt (24) wenigstens einer elektrischen Entladung (34) ausgesetzt wird.
Darüber hinaus wird eine Verwendung der erfindungsgemäßen Vorrichtung zur Detektion schwefelhaltiger und/oder chlorhaftiger und/oder stickstoffoxidhaltiger und/oder phosphorhaltiger gasförmiger Schadstoffe beansprucht.

## Beschreibung

Die Erfindung betrifft eine Vorrichtung und ein Verfahren zur Detektion gasförmiger Schadstoffe, wobei die gasförmigen Schadstoffe in wenigstens ein Umwandlungsprodukt zerlegt werden und der Nachweis des gasförmigen Schadstoffes anhand des wenigstens einen Umwandlungsproduktes erfolgt.

Es ist bekannt, zur Schädlingsbekämpfung von Gebäuden, auch einzelnen Ausstattungen von Gebäuden, sowie in Lebensmittel verarbeitenden Betrieben, beispielsweise in Lagerräumen, Transportbehältern (Container), Begasungsmittel einzusetzen. Durch den Einsatz dieser Begasungsmittel wird eine effektive Bekämpfung von Ungeziefer möglich, da das Begasungsmittel aufgrund der Gleichverteilung im Raum nahezu überall hingelangt, wo die zu bekämpfenden Ungeziefer sich aufhalten. Nach einer bestimmten Einwirkzeit des Begasungsmittels werden die mit dem Begasungsmittel beaufschlagten Räume und Gegenstände beziehungsweise Produkte entlüftet. Es kann jedoch nicht in jedem Fall sichergestellt werden, dass eine ausreichende Entlüftung erfolgt. Dies kann dazu führen, dass Reste des Begasungsmittels sich noch in den Gebäuden oder in Transportbehältern befinden. Personen, die dann in die Gebäude treten oder die Transportbehälter beispielsweise entleeren wollen oder kontrollieren wollen, wären somit einer Gesundheitsgefährdung ausgesetzt.

Als Begasungsmittel werden beispielsweise schwefelhaltige, chlorhaltige, stickstoffdioxydhaltige und/oder phosphorhaltige gasförmige Schadstoffe eingesetzt.

Messgeräte zur Detektion gasförmiger Stoffe sind bekannt. So beschreibt beispielsweise DE 37 29 286 C2 ein Messgerät zur Analyse eines Gasgemisches auf mindestens ein in sehr geringer Konzentration enthaltenes Gas, bei dem das Gasgemisch während der Messung mit einem geheizten Katalysatorelement in Kontakt gebracht wird, an dem das zu messende Gas in Umwandlungsprodukte zerlegt wird und die Umwandlungsprodukte mittels eines Gassensors nachgewiesen werden, der im Messzustand mindestens eines der Umwandlungsprodukte aufnimmt, wobei sich eine Messgröße des Sensors ändert, und bei dem der Gassensor nach der Messung durch Aufheizen auf eine gegenüber dem Messzustand erhöhte Temperatur in einen Regenerierungszustand gebracht wird, in dem aufgenommene Umwandlungsprodukte wieder abgegeben werden.

Es sind auch allgemein Gassensoren bekannt, mittels denen direkt in Gasgemischen vorhandene Stoffe detektiert werden können. Diese bekannten Gassensoren besitzen keine

Selektivität für beispielsweise die Detektion von Begasungsstoffen in komplexen Gemischen mit anderen chemischen Verbindungen.

Der Erfindung liegt die Aufgabe zugrunde, ein Verfahren und eine Vorrichtung der gattungsgemäßen Art zu schaffen, mittels denen in einfacher Weise gasförmige Schadstoffe detektiert werden können, insbesondere auch dann, wenn diese in geringer Konzentration und auch in Gegenwart anderer Substanzen vorliegen.

Erfindungsgemäß wird diese Aufgabe durch eine Vorrichtung mit den in Anspruch 1 genannten Merkmalen gelöst. Dadurch, dass die Vorrichtung eine Einrichtung zur Zerlegung der zu detektierenden gasförmigen Schadstoffe in wenigstens ein Umwandlungsprodukt aufweist, wobei diese Einrichtung einen Reaktionsraum umfasst, innerhalb dem eine elektrische Entladung erzeugbar ist, wird vorteilhaft möglich, die gasförmigen Schadstoffe in das wenigstens eine Umwandlungsprodukt zu zerlegen, indem mittels der elektrischen Entladung erzeugte freie Radikale und Ionen mit dem gasförmigen Schadstoff reagieren oder die elektrische Entladung den gasförmigen Schadstoff fragmentieren lässt. Die erhaltenen Umwandlungsprodukte lassen sich sehr viel selektiver mit größerer Empfindlichkeit nachweisen.

Die Aufgabe wird ferner durch ein Verfahren mit den in Anspruch 12 genannten Merkmalen gelöst. Dadurch, dass der gasförmige Schadstoff beziehungsweise ein den gasförmigen Schadstoff enthaltendes Gasgemisch wenigstens einer elektrischen Entladung ausgesetzt wird, die zu einer Zerlegung in wenigstens ein Umwandlungsprodukt des gasförmigen Schadstoffes führt, wird vorteilhaft möglich, einen selektiven Nachweis auch schwierig zu detektierender gasförmiger Schadstoffe zu erhalten, da durch die Zerlegung in die Umwandlungsprodukte ein indirekter Nachweis über wenigstens eines der Umwandlungsprodukte genauer und selektiver möglich ist.

Insgesamt wird durch das erfindungsgemäße Verfahren möglich, eine erfindungsgemäße Vorrichtung bereitzustellen, die sich durch einen einfachen Aufbau auszeichnet, einen geringen Verbrauch an elektrischer Energie umfasst und auf geringem Bauraum untergebracht werden kann. So sind insbesondere auch batteriebetriebene Handgeräte einsetzbar, mittels denen schnell und flexibel ein Nachweis gasförmiger Schadstoffe in Gebäuden oder Transportbehältern (Container oder dergleichen) möglich ist.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Zeichnungen näher erläutert. Es zeigen:
- Figur 1: eine schematische Gesamtansicht zur Anwendung einer erfindungsgemäßen Vorrichtung;
- Figur 2: eine schematische Darstellung des erfindungsgemäßen Verfahrens;
- Figur 3: eine schematische Darstellung der erfindungsgemäßen Vorrichtung;
- Figuren: idealisierte Signalverläufe der mit der erfindungsgemäßen Vorrichtung gewonnenen
- 4 und 5: Signale und
- Figuren: einige der bestehenden Kombinationsmöglichkeiten in schematischer
- 6 bis 8: Darstellung.

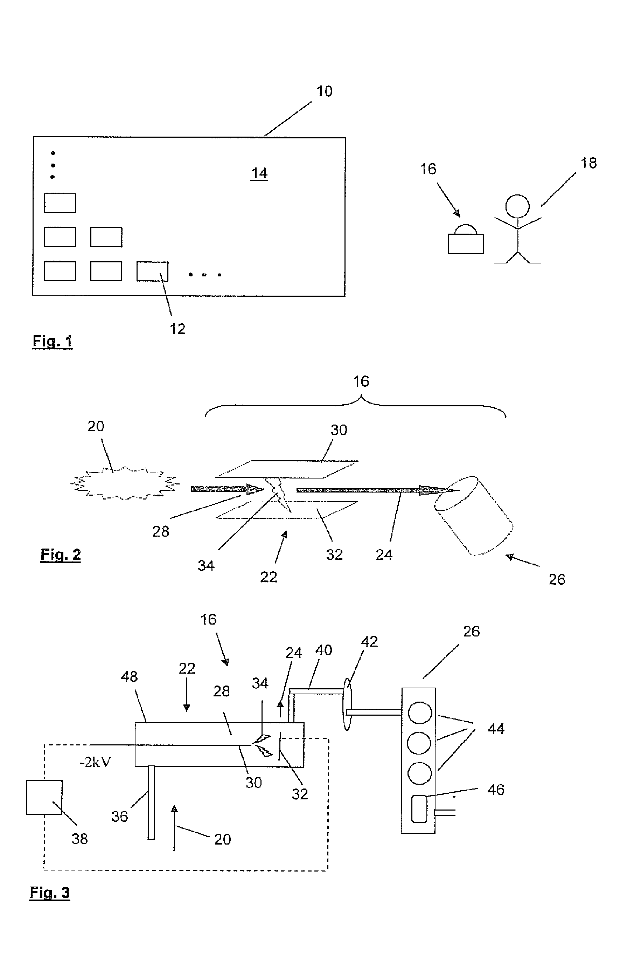

Figur 1 zeigt schematisch einen Transportbehälter 10, beispielsweise einen Überseecontainer. Innerhalb des Transportbehälters 10 sind hier angedeutet verpackte oder unverpackte Waren 12 transportiert. Der Transportbehälter 10 umfasst einen Innenraum 14, der mit einem Begasungsmittel, beispielsweise Sulfuryldifluorid (SO₂F₂), behandelt wurde. Nach Ankunft des Transportbehälters 10 an seinem Bestimmungsort soll überprüft werden, ob der Innenraum 14 ordnungsgemäß entlüftet wurde, das heißt keine flüchtigen Bestandteile des Begasungsmittels mehr vorhanden sind. Erst wenn dies festgestellt ist, kann eine Entladung oder Kontrolle der Waren 12 erfolgen.

Die Überprüfung erfolgt mittels einer Vorrichtung 16, die durch eine Bedienperson 18 handhabbar ist. Die Vorrichtung 16 ist ein handtragbares Messgerät, das beispielsweise mittels einer Messlanze in Verbindung mit dem Innenraum 14 des Transportbehälters 10 bringbar ist. Die Messung erfolgt unmittelbar und ist sofort an einem Display oder dergleichen der Vorrichtung 16 für die Bedienperson 18 ablesbar.

Figur 2 verdeutlicht schematisch das erfindungsgemäße Verfahren. Das im Innenraum 14 befindliche Gasgemisch 20, das den zu detektierenden gasförmigen Schadstoff enthält, wird der Vorrichtung 16 zugeführt. Die Vorrichtung 16 umfasst eine Einrichtung 22 zur Zerlegung der zu detektierenden gasförmigen Schadstoffe in wenigstens ein Umwandlungsprodukt. Dieses Umwandlungsprodukt 24 wird einer Einrichtung 26, beispielsweise einer elektrochemischen Messzelle, zur selektiven Bestimmung des wenigstens einen Umwandlungsproduktes 24 zugeführt. Die Einrichtung 22 umfasst einen Reaktionsraum 28, innerhalb dem zwischen zwei Elektroden 30 und 32 eine elektrische Entladung 34, vorzugsweise bei hohen Spannungen und geringen Strömen, das heißt eine Koronaentladung 34, erzeugt wird. Diese elektrische Entladung 34 führt zur Umwandlung des gasförmigen Schadstoffes in das wenigstens eine Umwandlungsprodukt 24.

Figur 3 zeigt schematisch den Aufbau der Vorrichtung 16. Gleiche Teile wie in den vorhergehenden Figuren sind mit gleichen Bezugszeichen versehen und nicht nochmals erläutert. So besitzt die Vorrichtung 16 die Einrichtung 22, die den Reaktionsraum 28 ausbildet. Der Reaktionsraum 28 ist über einen Einlass 36 mit der Umgebung verbunden. Über den Einlass 36 kann das Gasgemisch 20 in den Reaktionsraum 28 gelangen. Innerhalb des Reaktionsraumes 28 sind die Elektroden 30 und 32 angeordnet. Die Elektrode 30 ist hierbei stabförmig ausgebildet, die Elektrode 32 ist plattenförmig ausgebildet. Die Elektroden 30 und 32 sind wie angedeutet mit einer Spannungsversorgungsschaltung 38 verbunden. Durch Anlegen einer Spannung zwischen den Elektroden 30 und 32 - wie später noch näher erläutert - kommt es zur Ausbildung der Koronaentladung 34. Diese führt dazu, dass wenigstens ein Umwandlungsprodukt 24 entsteht, dass über einen Auslass 40 auf ein Filter 42 geführt wird. Das Filter 42, das lediglich optional vorhanden ist, gestattet eine selektive Filtration aus dem aus dem Ausgangsanschluss 40 austretenden Gasgemisch, das wenigstens ein Unwandlungsprodukt 24 enthält. Das Filter 42 kann so gewählt sein, dass nur dieses eine Umwandlungsprodukt 24 durchgelassen wird. Hinter dem Filter 42 befindet sich die Einrichtung 26, die wenigstens eine, im gezeigten Beispiel drei, Gassensoren, zum Beispiel drei unterschiedliche elektrochemische Messzellen 44 umfasst. Die Einrichtung 26 kann ferner einen Fotoionisationsdetektor 46 oder, wie nicht dargestellt, ein optisches Spektrometer oder ein Ionenmobilitätsspektrometer enthalten. Mit dem optischen Spektrometer lassen sich neben den Umwandlungsprodukten auch die angeregten Atome oder Moleküle direkt in der Gasentladung nachweisen.

Die in Figur 3 dargestellte Vorrichtung 16 zeigt folgende allgemeine Funktion:
Über den Einlass 36 wird das Gasgemisch 20, beispielsweise aus dem Innenraum 14 des Transportbehälters 10, angesaugt. Dieses gelangt in den Reaktionsraum 28 und tritt über den Auslass 40 aus diesem wieder aus. Innerhalb des Reaktionsraumes 28 wird zwischen den Elektroden 30 und 32 wenigstens eine Koronaentladung 34 erzeugt. Hierdurch findet eine Modifizierung von Bestandteilen des Gasgemisches statt. Enthält das Gasgemisch beispielsweise Sulfuryldifluorid (SO₂F₂), wird dieses durch die Koronaentladung in Schwefeldioxid (SO₂) und weitere Fragmente oder Produkte zersetzt. Zusätzlich kann durch die Gasentladung beziehungsweise Koronaentladung bei Anwesenheit von Luft Ozon oder/und Stickoxide entstehen. Diese Umwandlungsprodukte sind dann Bestandteil des Gasgemisches 20 und gelangen über den Auslass 40 auf das Filter 42. Das Filter 42 ist beispielsweise nur selektiv für das Umwandlungsprodukt Stickstoffdioxid (NO₂) oder Ozon (O₃), so dass diese nicht auf die Einrichtung 26 gelangen. Mittels einer der elektrochemischen Messzellen 44 und/oder des Fotoionisationsdetektors 46 kann ermittelt werden, ob das Gasgemisch 20 Schwefeldioxid (SO₂) enthält und in welcher Konzentration dieses vorhanden ist. Anhand dieses Messergebnisses kann dann durch die Bedienperson 18 erkannt werden, ob in dem Innenraum 14 Sulfuryldifluorid (SO₂F₂) vorhanden ist, und entsprechende Maßnahmen ergriffen beziehungsweise eingeleitet werden.

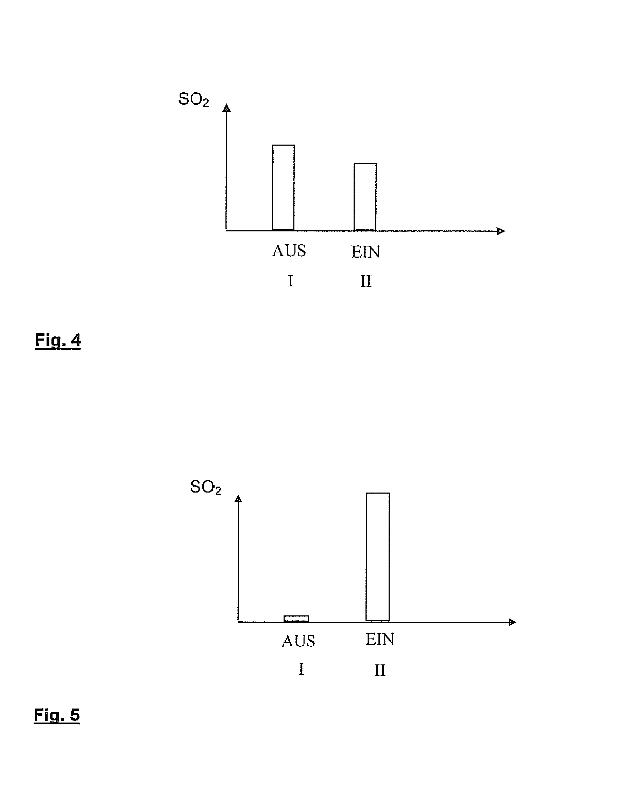

Um mittels der Vorrichtung 16 unterscheiden zu können, ob das Gasgemisch 20 eventuell selber schon Schwefeldioxid (SO₂) enthält oder Schwefeldioxid (SO₂) erst durch die Koronabehandlung von Sulfuryldifluorid (SO₂F₂) entstanden ist, sind verschiedene Betriebsmodi der Vorrichtung 16 möglich. So kann eine Messung in einem Betriebsmodus 1 erfolgen, bei dem die Koronaentladung 34 nicht aktiv ist. In einem Betriebsmodus II dagegen ist die Koronaentladung 34 aktiv. In Figur 4 und 5 sind idealisierte Signalverläufe, die sich bei den Betriebsmodi 1 beziehungsweise II ergeben können, dargestellt.

Figur 4 verdeutlicht, dass bei einem Betriebsmodus I, das heißt, die Koronaentladung 34 ist nicht aktiv, mittels der Einrichtung 26 dennoch Schwefeldioxid (SO₂) detektiert wird. Bei anschließendem Betriebsmodus II, das heißt, die Koronaentladung 34 ist aktiv, wird ebenfalls Schwefeldioxid (SO₂) detektiert. Bei einem derartigen Messergebnis wird deutlich, dass das Gasgemisch 20 zwar Schwefeldioxid (SO₂) jedoch kein Sulfuryldifluorid (SO₂F₂) enthält. Erkennbar wird dies an der Detektion des Schwefeldioxides (SO₂) während des Betriebsmodus I und dem etwas geringeren Niveau des detektierten Schwefeldioxid (SO₂) während des Modus II. Durch die Koronaentladung 34 kommt es zu einem geringfügigen Abbau von Schwefeldioxid (SO₂) innerhalb des Gasgemisches 20.

Figur 5 verdeutlicht die Situation, dass innerhalb des Gasgemisches 20 Sulfuryldifluorid (SO₂F₂) vorhanden ist. Während des Betriebsmodus I, das heißt, die Koronaentladung 34 ist nicht aktiv, wird kein beziehungsweise nur sehr wenig Schwefeldioxid (SO₂) detektiert. Beim anschließenden Betriebsmodus II, das heißt, die Koronaentladung 34 ist aktiv, wird Schwefeldioxid (SO₂) im größeren Umfange nachgewiesen. Dies ist ein Indiz dafür, dass durch die Koronaentladung 34 eine Zersetzung von Sulfuryldifluorid (SO₂F₂) in das Umwandlungsprodukt Schwefeldioxid (SO₂) erfolgte, das dann durch die Einrichtung 26 nachgewiesen wurde. In diesem Fall ist davon auszugehen, dass innerhalb des Transportbehälters 10 Sulfuryldifluorid (SO₂F₂) vorhanden ist und entsprechende Maßnahmen ergriffen werden müssen.

Zum Zuführen des Gasgemisches 20 in die Einrichtung 16 kann zusätzlich eine Fördereinrichtung, beispielsweise eine Pumpe oder dergleichen, vorgesehen sein, mittels der das Gasgemisch 20 angesaugt wird.

Gemäß der Darstellung in Figur 3 ist die stabförmige Elektrode 30 als Katode geschaltet und die großflächige Elektrode 32 als Anode. Das den Reaktionsraum 28 ausbildende Gehäuse 48 ist elektrisch geschirmt ausgebildet. Nach weiteren, nicht dargestellten Ausführungsbeispielen, kann die Katode auch von der großflächigen Elektrode 32 und die Anode von der stabförmigen Elektrode 30 gebildet sein. Auch sind Anordnungen denkbar, bei denen beide Elektroden 30, 32 stabförmig oder großflächig ausgebildet sind oder die großflächige Elektrode plattenförmig oder konusförmig gestaltet wird..

Die von der Spannungsversorgungsschaltung 38 bereitgestellte Spannung zur Ausbildung der elektrischen Entladung 34 kann ebenfalls unterschiedlich gewählt sein. So kann zwischen Elektroden 30 und 32 beispielsweise eine kontinuierliche Gleichhochspannung angelegt sein. Denkbar ist, die Gleichhochspannung auch pulsförmig anzulegen. Schließlich kann die elektrische Entladung 34 auch durch eine kontinuierlich angelegte Wechselhochspannung erzeugt werden.

Das Filter 42 kann beispielsweise einen Katalysator umfassen, der beispielsweise Nickeloxid, Süizium-Bor-Nitrid oder andere katalytisch aktive Substanzen aufweist, mittels denen beispielsweise selektiv Stickoxide NO₂, Schwefeldioxid SO₂ oder Ozon O₃ aus einer Entladung bei Luft als Trägergas gefiltert werden kann. An Stelle des Katalysators kann der Filter auch einen mit Chemikalien imprägnierten Träger umfassen, mittels dem die selektive Filterung erfolgen kann. Ferner kann das Filter 42 auch eine Heizquelle umfassen, mittels der die entsprechende selektive Filterung thermisch labiler Verbindungen erfolgen kann.

In Figur 3 ist beispielhaft auf die Einrichtung 26 eingegangen worden, die elektrochemische Messzellen 44 und einen Fotoionisationsdetektor 46 aufweisen kann. Denkbar ist auch der Einsatz von Metalloxidsensoren, Pellistoren und/oder Chemolumineszensdetektoren. Denkbar ist, dass die Einrichtung 26 mehrere gleichartige oder auch mehrere Gruppen gleichartiger Detektoren umfassen kann, mittels denen das gleiche Umwandlungsprodukt 24 mehrfach oder verschiedene Umwandlungsprodukte gleichzeitig detektiert werden können.

Denkbar ist auch der Einsatz von Gaschromotographen, optischen Spektrographen, welche die optischen Emissionslinien direkt in der Gasentladung und/oder die Chemolumineszensspektren nach der Gasentladung analysieren.

Nach dem Ausführungsbeispiel der Figur 3 umfasst die Vorrichtung 16 eine Einrichtung 22 zur Zerlegung des Gasgemisches 20 und eine Einrichtung 26 zur selektiven Bestimmung von Bestandteilen des Gasgemisches beziehungsweise daraus entstandenen Umwandlungsprodukten. Nach weiteren Ausführungsbeispielen kann die Vorrichtung 16 auch mehrere Einrichtungen 22 umfassen, die parallel geschaltet sind. So kann das Gasgemisch 20 zeitgleich in zwei oder mehr Einrichtungen 22 in Umwandlungsprodukte zerlegt werden. Hierbei ist denkbar, jede dieser Einrichtungen 22 mit einer eigenen Spannungsversorgungsschaltung 38 zu versehen, so dass unterschiedliche elektrische Entladungen 34 erzeugbar sind. Diese mehreren Einrichtungen 22 können dann beispielsweise auf ein gemeinsames Filter 42 und eine Einrichtung 26 geschaltet sein. In einem weiteren Ausführungsbeispiel kann jedoch auch jeder der parallel geschalteten Einrichtungen 22 ein eigenes Filter 42 und eine eigene Einrichtung 26 zugeordnet sein. Hierdurch kann die Vorrichtung 16 gleichzeitig selektiv auf mehrere zu detektierende Substanzen ausgelegt werden. Mit einem Messvorgang können dann gleichzeitig auch unterschiedliche Substanzen detektiert werden. Gleichzeitig ist eine Optimierung der jeweiligen Einrichtung 22 auf die zu detektierende Substanz möglich, indem die Einrichtung 22 beispielsweise unterschiedliche Elektrodengeometrien, unterschiedliche Spannungsversorgungen und dergleichen aufweist. Auch sind die Einrichtungen 26 entsprechend ihrer Detektoren, das heißt elektrochemische Messzellen, Metalloxidsensoren, Pellistoren, Chemolumineszensdetektoren, Fotoionisationsdetektoren, lonenmobilitätsspektrometer, optischen Spektrometern, Gaschromatograph und dergleichen, unterschiedlich ausstattbar, so dass sehr komplexe Messungen zeitgleich mit hoher Selektivität und Genauigkeit durchgeführt werden können. Insbesondere erhält man durch die Kombination der verschiedenen Detektoren zusätzliche Informationen, die eine Identifikation der chemischen Verbindungen ermöglichen, so ist zum Beispiel neben der Kombination der Vorrichtung 16 mit einfachen elektrochemischen Zellen auch eine Kombination mit komplexeren Detektoren, wie zum Beispiel einem optischen Spektrometer, zum Nachweis der angeregten Atome und Moleküle in der Gasentladung, und einem Ionenmobilitätsspektrometer, zum Nachweis der Moleküle ohne und mit Gasentladung, möglich.

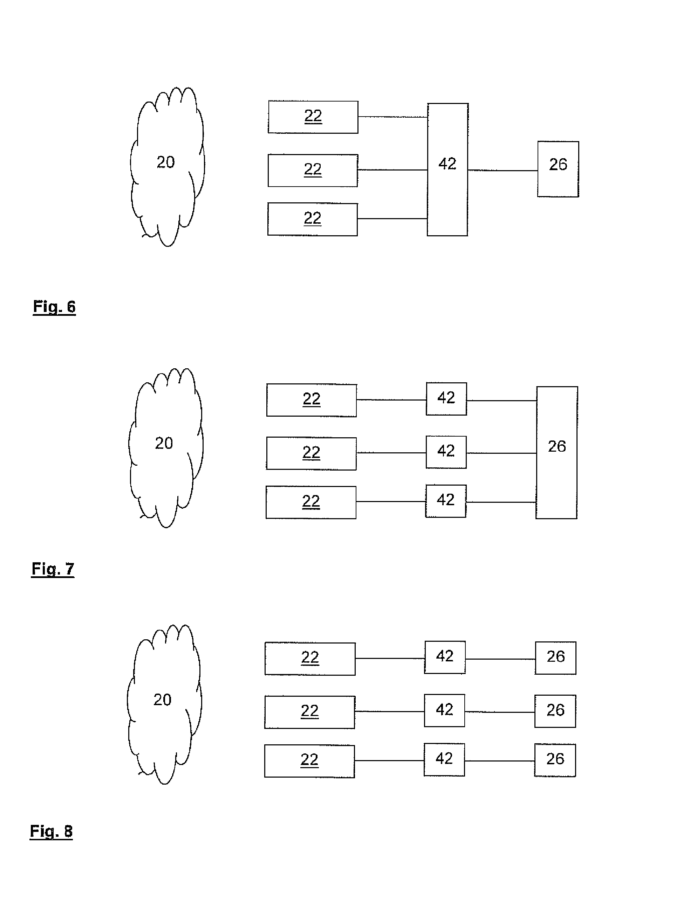

In den Figuren 6 bis 8 sind schematisch einige der bestehenden Kombinationsmöglichkeiten dargestellt.

Als Trägergas für die elektrischen Entladungen 34 innerhalb des Gehäuses 48 kann beispielsweise atmosphärische Luft, Helium, Argon oder Wasserstoff eingesetzt werden.

Die erfindungsgemäße Einrichtung kann zur Detektion schwefelhaltiger Gefahrstoffe eingesetzt werden, indem eine Zerlegung in Schwefeldioxid SO₂ oder andere schwefelhaltige Verbindungen erfolgt. Es kann auch eine Detektion von chlorhaltigen Gefahrstoffen, wie beispielsweise Chlorpikrin erfolgen, indem eine Zerlegung in die Umwandlungsprodukte Chlorwasserstoff oder andere Chlorverbindungen erfolgen. Auch ist die Detektion von stickstoffdioxidhaltigen Gefahrstoffen wie beispielsweise bei Sprengstoffen möglich, indem der Nachweis über die Detektion von Stickoxiden NO, NO₂ oder anderen Stickstoffverbindungen erfolgt. Schließlich ist auch die Detektion phosphorhaltiger Gefahrstoffe, wie beispielsweise Pestizide, möglich, indem der Nachweis über phosphorhaltige Umwandlungsprodukte erfolgt.

### Bezugszeichenliste

- 10: Transportbehälter
- 12: Waren
- 14: Innenraum
- 16: Vorrichtung zur Überprüfung
- 18: Bedienperson
- 20: Gasgemisch
- 22: Einrichtung zur Zerlegung
- 24: Umwandlungsprodukt
- 26: Einrichtung zur selektiven Bestimmung
- 28: Reaktionsraum
- 30: Elektrode
- 32: Elektrode
- 34: Entladung
- 36: Einlass
- 38: Spannungsversorgungsschaltung
- 40: Auslass
- 42: Filter
- 44: elektrochemische Messzellen
- 46: Fotoionisationsdetektor
- 48: Gehäuse

- I: Betriebsmodus, Koronaentladung nicht aktiv
- II: Betriebsmodus, Koronaentladung aktiv

## Patentansprüche

1. Vorrichtung (16) zur Detektion gasförmiger Schadstoffe mit einer Einrichtung (22) zur Zerlegung der zu detektierenden gasförmigen Schadstoffe in wenigstens ein Umwandlungsprodukt (24) und einer Einrichtung (26) zur selektiven Bestimmung des wenigstens einen Umwandlungsproduktes (24), **dadurch gekennzeichnet, dass** die Einrichtung (22) zur Zerlegung des zu detektierenden gasförmigen Schadstoffes wenigstens einen Reaktionsraum (28) umfasst, innerhalb dem eine elektrische Entladung (34) erzeugbar ist.

2. Vorrichtung nach Anspruch 1, **dadurch gekennzeichnet, dass** die Einrichtung (22) über ein Filter (42) mit der Einrichtung (26) verbunden ist.

3. Vorrichtung nach einem der vorhergehenden Ansprüche, dadurch geikennzeichnet, dass innerhalb des Reaktionsraumes (28) zwei Elektroden (30, 32) angeordnet sind, die mit einer Spannungsversorgungsschaltung (38) verbunden sind.

4. Vorrichtung nach einem der vorhergehenden Ansprüche, **dadurch gekennzeichnet, dass** die Elektroden (30, 32) stabförmig und/oder großflächig ausgebildet sind.

5. Vorrichtung nach einem der vorhergehenden Ansprüche, **dadurch gekennzeichnet, dass** die großflächigen Elektroden (30, 32) plattenförmig oder konusförmig ausgebildet sind.

6. Vorrichtung nach einem der vorhergehenden Ansprüche, dadurch ge6cennzeichnet, dass die Einrichtung (22) mehrere, parallel geschaltete Reaktionsräume (28) umfasst.

7. Vorrichtung nach einem der vorhergehenden Ansprüche, **dadurch gekennzeichnet, dass** jeder der Reaktionsräume (28) über ein Filter (42) mit der Einrichtung (26) verbunden ist.

8. Vorrichtung nach einem der vorhergehenden Ansprüche, **dadurch gekennzeichnet, dass** die mehreren Reaktionsräume (28) über ein gemeinsames Filter mit der Einrichtung (26) verbunden sind.

9. Vorrichtung nach einem der vorhergehenden Ansprüche, **dadurch gekennzeichnet, dass** die Einrichtung (26) wenigstens zwei Detektoren zur Bestimmung des wenigstens einen Umwandlungsproduktes (24) umfasst.

10. Vorrichtung nach einem der vorhergehenden Ansprüche, **dadurch gekennzeichnet, dass** die Einrichtung (26) wenigstens eine elektrochemische Messzelfe (44) und/oder wenigstens einen Fotoionisationsdetektor (46) und/oder wenigstens ein optisches Spektrometer und/oder wenigstens ein Ionenmobilitätsspektrometer und/oder wenigstens einen Metalloxidsensor und/oder wenigstens einen Pellistor und/oder wenigstens einen Chemolumineszensdetektor und/oder wenigstens einen Gaschromotographen umfasst.

11. Vorrichtung nach einem der vorhergehenden Ansprüche, **dadurch gekennzeichnet, dass** die Einrichtung (26) wenigstens ein optisches Spektrometer umfasst, welches die optische Emission in der Gasentladung beobachtet und/oder wenigstens eine elektrochemische Messzelle (44) und/oder wenigstens einen Fotoionisationsdetektor (46) und/oder wenigstens ein Ionenmobilitätsspektrometer und/oder wenigstens einen Metalloxidsensor und/oder wenigstens einen Pellistor und/oder wenigstens einen Chemolumineszensdetektor und/oder wenigstens einen Gaschromotographen umfasst.

12. Verfahren zur Detektion gasförmiger Schadstoffe, wobei der gasförmige Schadstoff in wenigstens ein Umwandlungsprodukt (24) zerlegt wird, **dadurch gekennzeichnet, dass** der zu detektierende gasförmige Schadstoff zur Zerlegung in das wenigstens eine Umwandlungsprodukt (24) wenigstens einer elektrischen Entladung (34) ausgesetzt wird.

13. Verfahren nach Anspruch 12, **dadurch gekennzeichnet, dass** die elektrische Entladung (34) als Koronaentladung erzeugt wird.

14. Verfahren nach einem der Ansprüche 12 und 13, **dadurch gekennzeichnet, dass** die elektrische Entladung (34) mittels einer Hochgleichspannung erzeugt wird.

15. Verfahren nach einem der Ansprüche 12 bis 14, **dadurch gekennzeichnet, dass** die elektrische Entladung (34) mittels einer Hochwechselspannung erzeugt wird.

16. Verwendung der Vorrichtung nach wenigstens einem der Ansprüche 1 bis 11 zur Detektion schwefelhaltiger und/oder chlorhaltiger und/oder stickstoffoxidhaltiger und/oder phosphorhaltiger gasförmiger Schadstoffe.
